# EUROPEAN PATENT SPECIFICATION

(11) **EP 2 470 066 B1**
(45) Date of publication and mention of the grant of the patent: **23.10.2013**
(21) Application number: 10768281.7
(22) Date of filing: 27.08.2010
(51) Int. Cl.: A61B 5/00, A61B 5/026, A61B 5/083

(54) **A NON-INVASIVE METHOD FOR ASSESSING TISSUE PERFUSION IN A PATIENT**
NICHT-INVASIVES VERFAHREN ZUR UNTERSUCHUNG DER GEWEBEPERFUSION BEI EINEM PATIENT
PROCÉDÉ NON INVASIF D'ÉVALUATION DE LA PERFUSION TISSULAIRE CHEZ UN PATIENT

(30) Priority: 28.08.2009 WO PCT/IB2009/006903
(43) Date of publication of application: 04.07.2012
(73) Proprietor: Assistance Publique - Hôpitaux de Paris, 75004 Paris (FR)
(72) Inventor: VALLEE, Fabrice, F-75019 Paris (FR); MATEO, Joaquim, F-75018 Paris (FR); PAYEN DE LA GARANDERIE, Didier, F-75018 Paris (FR)
(74) Representative: Marcadé, Véronique
(86) International application number: PCT/IB2010/002407
(87) International publication number: WO 2011/024081

(56) References cited:
- ROOTH, G. , EWALD, U. AND CALIGERA, F.: "Transcutaneous PO2 and PCO2 monitoring at 37 degrees Celsius cutaneaous PO2 and PCO2", ADV. EXP. MED. BIOL., vol. 220, 1987, pages 23-32, XP009131627, cited in the application
- YAKOV SIVAN ET AL.: "Estimation of Arterial Carbon Dioxide by End-Tidal and Transcutaneous PCO2 Measurements in Ventilated Children", PEDIATRIC PULMONOLOGY, vol. 12, no. 3, March 1992 (1992-03), pages 153-157, XP002581196,
- MAURO MANISCALCO ET AL: "Evaluation of a transcutaneous carbon dioxide monitor in severe obesity", INTENSIVE CARE MEDICINE, SPRINGER, BERLIN, DE, vol. 34, no. 7, 26 March 2008 (2008-03-26) , pages 1340-1344, XP019619062, ISSN: 1432-1238

## Description

Several clinical situations, such as sepsis, hemorrhage and cardiac arrest lead to a decrease of tissue perfusion. Consequently, the carbon dioxide resulting from metabolism is not carried away as in healthy states, and the partial pressure of carbon dioxide increases.

Cardiovascular resuscitation in septic shock received recently more attention both for macro- and microcirculation parameters. If early goal optimization is recommended during the so called "golden hours" on the basis of macro-circulatory parameters (Dellinger et al., 2008), some patients continue to have compromised tissue perfusion in relation with microcirculatory disturbances. It has been clearly shown using different techniques that tissue microperfusion can be impaired in septic shock with low skin Laser Doppler blood flow (Neviere et al., 1996), reduction in sublingual small vessels perfusion using SDF (De Backer et al., 2002), or abnormal post-occlusion tissue hemoglobin saturation using NIRS (Creteur et al., 2007). During the last decade, gastric tonometry became popular to assess gastric tissue perfusion as an index of splanchnic perfusion. Some studies showed clearly the relation between high gastric PCO₂ and persistence of hypoperfusion in life threatening conditions, including septic shock (Gutierrez et al., 1992; Heino et al., 1998; Levy et al., 2003a). Because of the price, the relatively invasive technique and the debate on the conditions to perform measurements (with or without an H+ pump inhibitor), such a device is not widely used.

However, the concept of an increase in tissue CO₂ during stagnant perfusion remains relevant as shown by publications of Weil and colleagues (Fries et al., 2006; Weil, 2000; Weil and Sun, 2001). These authors clearly demonstrated the elevation in tissue CO₂ during experimental hemorrhagic (Jin et al., 1998; Ristagno et al., 2006) or septic shock (Fang et al., 2006). When systemic and regional blood flow decreases, a CO₂ accumulation can be detected by tonometry earlier than other hypoperfusion parameters such as lactate level (Marik and Bankov, 2003). This parameter seems also suitable to assess the reperfusion effectiveness during resuscitation. Because of these studies, simple sublingual devices have been proposed to monitor tissue CO₂ level. However, this device remains expensive and uncomfortable for the patient, especially if it is to be used for a continuous monitoring.

In the past, especially for pediatric patients for whom invasive technique is not adequate, intensivists have developed the use of transcutaneous CO₂ measurements to monitor the adequacy of ventilation. This technique imposes skin warming by the electrode to "arteriolize" capillary blood for PCO₂ measurements, so that the measured PCO₂ is representative of the arterial PCO₂ (Eberhard, 2007; Mindt et al., 1982). In practice, the sensor is heated at 42°C to 44°C, and up to 45°C (Kagawa et al., 2004) prior to its application on the skin.

Transcutaneous PCO₂ sensors have also been proposed for measuring metabolic acidosis, especially in sport or occupational medicine (Rooth et al., 1987). In this publication, the authors use the sensors at 37°C, so that the measured PCO₂ is the cutaneous rather than the transcutaneous PCO₂.

The inventors have now demonstrated that, contrarily to the currently accepted paradigm, it is possible to assess tissue perfusion, with a good reactivity, by measuring PCO₂ using a fully non-invasive technique at the skin level, and by subtracting to this measure the arterial PCO₂ or another measure representative thereof, such as the end-tidal PCO₂. Indeed, as shown in the experimental data below, the use of a cutaneous PCO₂ sensor, without heating it at a temperature superior to 37°C, gives reliable information about tissue perfusion in critically ill or injured patients. Moreover, this information can be used as a prognostic marker in certain conditions, especially in septic shock.

In the present text, the following definitions and notations will be used:
aPCO₂ is the arterial partial pressure of carbon dioxide.
EtPCO₂ means the end-tidal partial pressure of carbon dioxide.

Both arterial and end-tidal PCO₂ can be measured by any technique known by the skilled artisan.

TcPCO₂ is the transcutaneous partial pressure of carbon dioxide, i.e., the partial CO₂ pressure measured with a sensor applied to the skin surface in conditions where the tissue underneath the sensor surface is arterialized, which means, in practice, that the sensor is heated at a temperature of 42°C to 44°C. Tanscutaneous partial pressure of carbon dioxide has been used for almost 40 years, especially in infants and premature neonates, as a surrogate measure of arterial PCO₂.

cPCO₂ is the cutaneous partial pressure of carbon dioxide, measured with a PCO₂ sensor applied to the skin surface in absence of arterialization of the tissue beneath the sensor surface. In practice, this means that the warming of the sensor is limited to normal central temperature (37°C or at most 37.5°C) or less (ambient or skin temperature).

A first aspect of the present invention is the use of [cPCO₂ - aPCO₂] or [cPCO₂ - EtPCO₂], as an indicator of tissue perfusion in a patient, wherein [cPCO₂ - aPCO₂] is the difference between cutaneous and arterial partial pressures of carbon dioxide of said patient, and [cPCO₂ - EtPCO₂] is the difference between cutaneous and end-tidal partial pressures of carbon dioxide of said patient. In particular, [cPCO₂ - aPCO₂] and [cPCO₂ - EtPCO₂] enable the *ex vivo* assessment of tissue microperfusion, *i.e.*, both macro- and microperfusion.

According to the present invention, [cPCO₂ - aPCO₂] and [cPCO₂ - EtPCO₂] during adequate ventilation also constitute markers which can be used by physicians for assessing impairment of blood circulation of a patient. Indeed, when [cPCO₂ - aPCO₂] or [cPCO₂ - EtPCO₂] are greater than predetermined thresholds, this indicates that the patient undergoes perfusion failure. As described in the experimental part below, the inventors have determined that when using their measurement techniques, [cPCO₂ - aPCO₂] ≥ 9 mm Hg, or [cPCO₂ - EtPCO₂] ≥ 15 mm Hg,is indicative of perfusion failure. Of course, these thresholds could be modified in the future, especially if different techniques are used for measuring the carbon dioxide partial pressures (for example, if cPCO₂ is measured at a different locus or by a different type of sensor). Performance of the described method on a larger amount of patients will also enable to refine these thresholds for detecting perfusion failure, but it can be expected that the refined threshold for [cPCO₂ - aPCO₂] will stay over 7 mm Hg, and the refined threshold for [cPCO₂ - EtPCO₂] will stay over 12 mm Hg.

As mentioned above, PCO₂ sensors have been used for a long time for measuring TcPCO₂ as an indicator of aPCO₂, necessitating heating of the sensor at a temperature between 42°C to 45°C before applying it to the skin (Kagawa et al., 2004). To the contrary, according to the invention, the partial CO₂ pressure which is used is the cutaneous PCO₂. According to a specific embodiment of the invention, cPCO₂ is measured with a transcutaneous PCO₂ sensor heated at a temperature ≤ 37°C, or applied to the skin without previous heating. Preferred locations for obtaining cPCO₂ according to the present invention are ear lobes and the scalp.

Another important aspect of the present invention is hence a method for assessing perfusion failure of a patient, comprising:
(i) placing a carbon dioxide sensor on the ear lobe or on the scalp of said patient, without previous heating of said sensor at a temperature significantly superior to 37°C (normal body temperature), i.e., without heating at all or with a heating at a temperature ≤ 37.5°C, preferably at ≤ 37°C, for example between 25 and 37°C;
(ii) measuring a partial pressure of carbon dioxide at the skin surface (cPCO₂);
(iii) measuring the arterial partial pressure of carbon dioxide (aPCO₂);
wherein [cPCO₂ - aPCO₂] ≥ 9 mm Hg is indicative of perfusion failure in the patient.

Alternatively, the above method can be performed by measuring the end-tidal pressure of carbon dioxide (EtPCO₂) instead of the arterial partial pressure; in this case, [cPCO₂ - EtPCO₂] ≥ 15 mm Hg is indicative of perfusion failure in the patient. In this embodiment, it is preferable to perform, in addition, a control of the arterial PCO₂, since the difference between aPCO₂ and EtPCO₂ depends on the patient's lung function, and hence may vary from one patient to another.

According to another embodiment, the invention pertains to the use of [cPCO₂ - EtPCO₂], for performing a continuous and non-invasive hemodynamic monitoring of a patient in a life-threatening condition. This difference is indeed a more sensitive marker than any other parameter described before, especially since in low pulmonary flow state, end-tidal PCO₂ decreases and tissue PCO₂ increases.

The invention hence also relates to a method for performing a continuous and non-invasive hemodynamic monitoring of a patient, comprising:
(i) placing a carbon dioxide sensor on the ear lobe or on the scalp of said patient, without previous heating of said sensor at a temperature superior to 37.5°C (*i.e.*, without heating at all or with a heating at a temperature ≤ 37.5°C, preferably at ≤ 37°C, for example between 25 and 37°C);
(ii) measuring a partial pressure of carbon dioxide at the skin surface (cPCO₂);
(iii) measuring the end-tidal partial pressure of carbon dioxide (EtPCO₂);
(iv) calculating [cPCO₂ - EtPCO₂],
wherein an increase of [cPCO₂ - EtPCO₂] indicates a deterioration of the hemodynamic state of the patient, and a decrease of [cPCO₂ - EtPCO₂] indicates an amelioration of the hemodynamic state of the patient.

When performing the above method, steps (ii) to (iv) can be performed continuously or punctually, with a frequency ranging from once a minute of once a day. As above-mentioned, the arterial partial pressure of carbon dioxide is also advantageously measured (to interprete EtPCO₂), for example once a day.

According to a particular aspect of the invention, [cPCO₂ - aPCO₂] or [cPCO₂ - EtPCO₂] is used for outcome prediction of a patient in intensive care unit. For example, for a patient in septic shock, a decrease of [cPCO₂ - aPCO₂] or [cPCO₂ - EtPCO₂] during the 24 hours following the onset of septic shock is indicative of a good prognosis, whereas the absence of decrease (steady state or increase) is indicative of a bad prognosis. More specifically, the inventors have demonstrated that in case of septic shock, [cPCO₂ - aPCO₂] and [cPCO₂ - EtPCO₂] must have decreased under certain thresholds, within the 24 hours following the onset of the treatment of septic shock, to be indicative of a good prognosis. Indeed, if [cPCO₂ - aPCO₂] ≥ 16 mm Hg, or if [cPCO₂ - EtPCO₂] ≥ 25 mm Hg 24 hours after the beginning of septic shock treatment, this is indicative of a bad prognosis (the patient is at high risk of death, its chances to recover from the shock are very poor).

Of course, the present invention also pertains to the *ex vivo* steps of the above methods, *i.e*., the calculation of [cPCO₂ - aPCO₂] and/or [cPCO₂ - EtPCO₂], followed by the interpretation of the obtained result(s) as described above.

Another aspect of the present invention is a device for performing a method as described above, comprising:
- a first carbon dioxide sensor for detecting a partial pressure of carbon dioxide (PCO₂), the sensor being adapted for being attached on the ear lobe and/or the scalp and measuring the cutaneous PCO₂ (cPCO₂) of a patient;
- a second carbon dioxide sensor for detecting a partial pressure of carbon dioxide (PCO₂), the sensor being adapted for measuring the end-tidal PCO₂ (EtPCO₂) of an intubated patient; and
- a computer operably connected to both sensors, wherein said computer calculates the difference between the partial pressures of carbon dioxide measured by said sensors.

The computer comprises at least calculator means, but it can also advantageously comprise a memory for stocking all the data of each patient, so that the history of the hemodynamic monitoring can be obtained for each patient.

According to a preferred embodiment, the device further comprises a third carbon dioxide sensor, said third sensor being adapted for measuring the arterial PCO₂ (aPCO₂). In this case, the computer calculates [cPCO₂ - aPCO₂] and [EtPCO₂ - aPCO₂]. Alternatively, the arterial PCO₂ is measured by means independent from the device, and the obtained value is entered in the computer *via* an interface. Another alternative is to use the first sensor to punctually measure transcutaneous PCO₂ (and hence, aPCO₂), by transiently warming said sensor.

The device according to the present invention can also comprise indicating means operably connected to the computer, wherein the indicating means indicate a degree of perfusion of the patient associated with the detected partial pressures of carbon dioxide. Optionally, the device also comprises a circuit for generating an alarm indicating a change of [cPCO₂ - EtPCO₂] with time. The computer can be programmed so that the signal sounds when [cPCO₂ - EtPCO₂] becomes higher than a predetermined value, for example 15 mm Hg to indicate perfusion failure. For a patient who already has a perfusion failure but for whom [cPCO₂ - EtPCO₂] is under 25 mm Hg, this threshold can be increased to 25 mm Hg, so that the signal rings if the patient's state worsens.

Other characteristics of the invention will also become apparent in the course of the description which follows of the experimental data obtained by the inventors and which provide it with the required experimental support, without limiting its scope.

### LEGENDS TO THE FIGURES

**Figure 1****:** Comparison of baseline value of [cPCO2 - aPCO2] and [cPCO2 - EtPCO2] between ICU-controls patients and septic shock patients. *, * *, * * * : p<0.05, 0.01, 0.001.
**Figure 2****:** ROC curves comparing the ability of [cPCO2 - aPCO2] (solid line) and [cPCO2 - EtPCO2] (dash line) to distinguish shock and control patients at baseline. ROC curve area: 0.95 (0.85-0.99) and 0.96 (0.86-0.99) for [cPCO2 - aPCO2] and [cPCO2 - EtPCO2], respectively. The best cut-off values were 9 mm Hg for [cPCO2 - aPCO2] and 15 mm Hg for [cPCO2 - EtPCO2].
**Figure 3****:** Evolution from H0 to H36 and comparison between survivors and non-survivors of Scv02 (%), Cardiac output (1/min), MAP (mmHg), CVP (mmHg), [cPCO2 - aPCO2] (mmHg), and [cPCO2 - EtPCO2] (mmHg). Expressed as mean ± SD.
**Figure 4****:** ROC curves comparing the ability of [cPCO2 - aPCO2] (solid line) and [cPCO2 - EtPCO2] (dash line) to distinguish survivors from non survivors at H24. ROC curve areas for [cPCO2 - aPCO2] and [cPCO2 - EtPCO2] were 0.85 (0.66-0.95) and 0.87 (0.69-0.96), respectively. A threshold of 16 mmHg for [cPCO2 - aPCO2] and 25 mmHg for [cPCO2 - EtPCO2] (dash line) discriminated survivors from non-survivors with a sensibility of 83% and a specificity of 90%.
**Figure 5****:** Relation between changes in [cPCO2 - aPCO2] and [cPCO2-EtPCO2] and changes in tissue microperfusion (delta TPU) assessed by laser Doppler during 16 fluid challenges.

### EXAMPLES

The aims of the present study were to: 1- propose the use a transcutaneous measurement of PCO₂ in normothermic condition (37°C) as a cutaneous PCO₂ measurement: (cPCO₂); 2- validate such measurement as an indicator of skin hypoperfusion in septic shock; 3- evaluate the interest of the gradient between arterial PCO₂ and cutaneous PCO₂ [cPCO₂ - aPCO₂] as a marker of hypoperfusion and therapeutic improvement; 4- propose a continuous non-invasive gradient between end tidal PCO₂ and cutaneous PCO₂ [cPCO₂ - EtPCO₂] as an alternative to evaluate the macro and microcirculation impact on cutaneous perfusion in septic shock-induced circulatory failure.

### Material and Methods

*Study population:* Thirty septic shock patients were studied within 24 hours after the onset of septic shock. Fifteen hemodynamically stable ICU patients without infection were studied and considered as control.

*Study Protocol:* For septic patients and "control" patients, following parameters were collected: central temperature, systemic hemodynamic and respiratory parameters, cutaneous PCO₂ (cPCO₂) and received treatment. Timing for data collection in septic patients was: just before inclusion (H0) and every 6 hours from H0 to H36 or until death. At each time, arterial and central venous blood samples were drawn for arterial and central venous blood gases analysis. Systemic hemodynamic data, blood samples and cPCO₂ were also collected just before and 10 min after performing a fluid challenge: a volume expansion of 500 ml saline or 250 ml of gelatin infused in 15 minutes.

*Cutaneous PCO₂ measurement:* Cutaneous PCO₂ was measured at ear lobe with TOSCA 500 monitor, (TOSCA®, Radiometer Copenhagen, Denmark)(Eberhard et al., 2002). The sensor was calibrated *in vitro*, and then fixed on the provided clip to be put at ear lobe after application of one drop of contact gel (Eberhard et al., 2002). After a few minutes for cPCO₂ value equilibration, measurements could be recorded. In the present study, the sensor was heated at 37°C instead of 42°C and the automatic correction (4 mm Hg) (Hazinski and Severinghaus, 1982) was not used.

*Carbon dioxide gradients:* [cPCO₂ - aPCO₂] and [cPCO₂ - EtPCO₂] gradients were calculated just before inclusion (H0) and every 6 hours from H0 to H36 or until death.

*Microcirculation blood flow assessment:* to separate eventual microcirculatory impairment from macrocirculation, the skin microcirculatory blood flow (mBFₛₖᵢₙ) was measured in 16 patients on the other ear lobe, at the same location as cPCO₂, using Laser Doppler (Transonic system HT107/HT207). Laser Doppler measurement was also performed during the fluid challenge as described above (Levy et al., 2003b).

### Results

### Comparison between septic shock and control patients

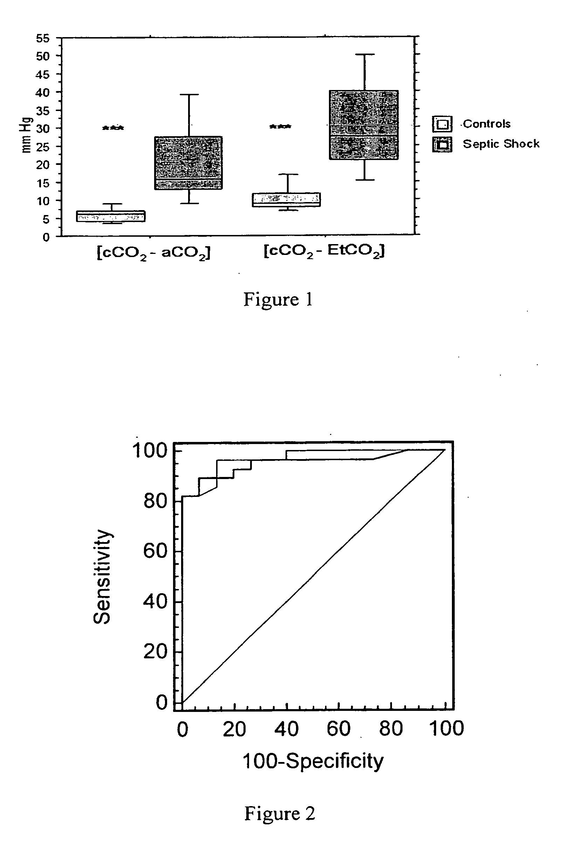

At baseline, [cPCO₂ - aPCO₂] and [cPCO₂ - EtPCO₂] were significantly higher in septic shock than in control patients: 20.3±11.1 versus 5.9±2.1mm Hg and 30±12 versus 10±4mmHg, p<0.0001, respectively (Fig. 1). Area under ROC curve was 0.95 (0.85-0.99) for [cPCO₂ - aPCO₂] and 0.96 (0.86-0.99) for [cPCO₂ - EtPCO₂],. A threshold of 9 mmHg for [cPCO₂ - aPCO₂] discriminated shock patients from control patients with a sensibility of 90% and a specificity of 93%. A threshold of 15 mmHg for [cPCO₂ - EtPCO₂] discriminated shock patients from control patients with a sensibility of 89% and a specificity of 87% (Fig. 2).

### [cPCO₂ - aPCO₂] and [cPCO₂ - EtPCO₂] evolution in septic shock patients

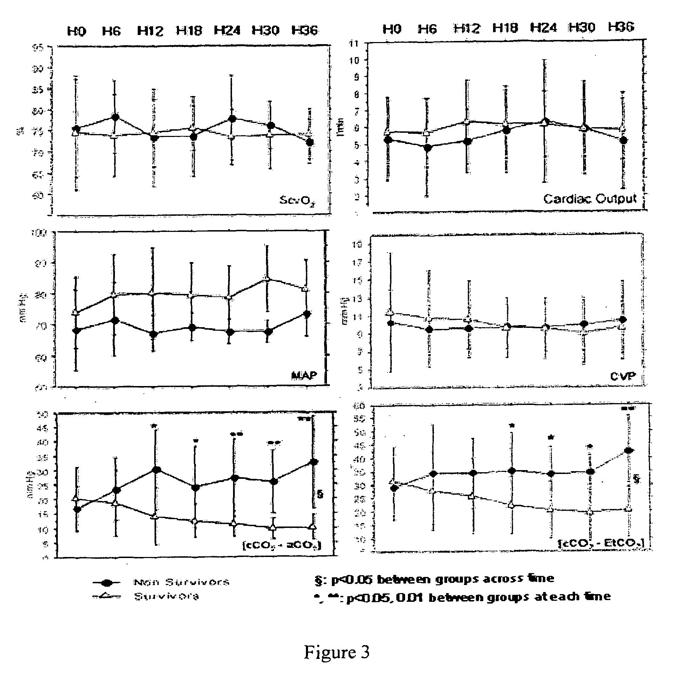

Among the 30 septic shock patients included, 23 (77%) survived and 7 died (23%). At baseline, there was no statistically significant difference for any hemodynamic parameters, gravity score and treatment received between survivors and non-survivors. During the next 36 hours of evolution, [cPCO₂ - aPCO₂] and [cPCO₂ - EtPCO₂] decreased significantly in survivors (20.2±11.1 to 10.2±4.3 mmHg and 31.3±12.8 to 20.3±10.3 mmHg, p<0.01, respectively). During the same time interval, the evolution of macro-hemodynamic parameters, such as mean arterial pressure (MAP), central venous pressure (CVP), cardiac output (CO) and central venous saturation (Scv02), was not different between survivors and non-survivors (Fig. 3).

### [cPCO₂ - aPCO₂] and [cPCO₂ - EtPCO₂] at H24 and relation with outcome

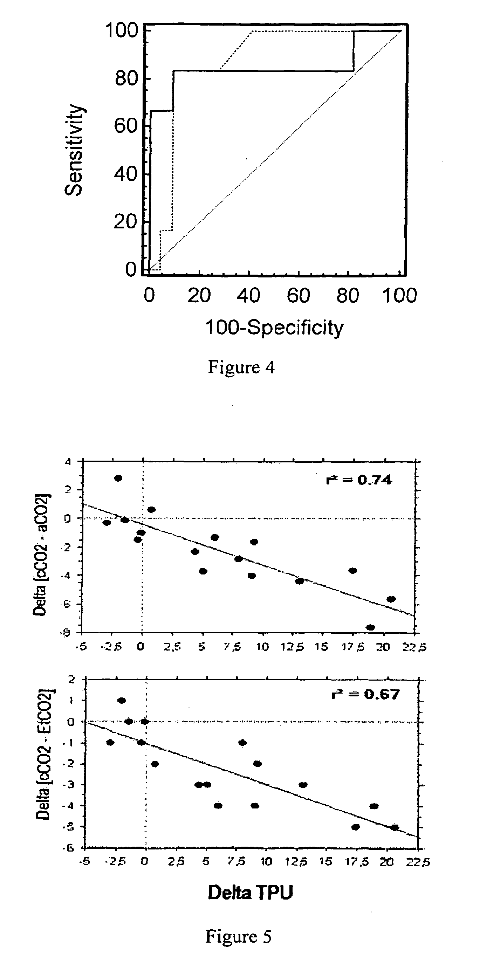

At H24, [cPCO₂ - aPCO₂] and [cPCO₂ - EtPCO₂] were significantly higher in non-survivors than in survivors: 27.3±13.5 versus 11.4±4.5mm Hg and 34±10 versus 20±10mmHg, p<0.01 respectively (Fig. 3). Areas under ROC curves for [cPCO₂ - aPCO₂] and [cPCO₂ - EtPCO₂] were 0.85 (0.66-0.95) and 0.87 (0.69-0.96), respectively. A threshold of 16 mmHg for [cPCO₂ - aPCO₂] and 25 mmHg for [cPCO₂ - EtPCO₂] discriminated survivors from non-survivors with a sensibility of 83% and a specificity of 90% (Fig. 4).

### [cPCO₂ - aPCO₂] and [cPCO₂ - EtPCO₂] acute variation during fluid challenges

In the 30 septic shock patients, 66 fluid challenges were performed. During fluid challenges, [cPCO₂ - aPCO₂] and [cPCO₂ - EtPCO₂] decreased significantly: from 14.5±7.2 to 12.7±7.2 mm Hg and from 23.4±8.7 to 21.7±8.5 mm Hg, p<0.001, respectively.

### Relationship to microcirculatory blood flow(mBFₛₖᵢₙ₎

In the 16 patients for whom mBFₛₖᵢₙ on the controlateral ear lobe was measured (tissular perfusion unit TPU (Schabauer and Rooke, 1994)), the fluid challenge increased significantly mBFₛₖᵢₙ :from 23±11 to 29±16 (26%), p<0.01. This increase in TPU strongly and inversely correlated with the decrease in either [cPCO₂ - aPCO₂] or [cPCO₂ - EtPCO₂]: r²=0.74, p<0.001 and r²=0.67, p<0.001, respectively (Fig. 5).

### Discussion

This study shows that: (1) a cut off-value of 9 mm Hg for the gradient [cPCO₂ - aPCO₂] measured at ear lobe at 37°C discriminates shocked and non-shocked patients with a sensibility of 90% and a specificity of 93%.(2) The evolution of such a gradient [cPCO₂ - aPCO₂] related to the prognosis of septic shock patients better than macro-circulation parameters. (3) [cPCO₂ - aPCO₂] during fluid challenge decreased in correlation with increase of mBFₛₖᵢₙ. (Laser Doppler). (4) These results were also observed using a strictly non invasive and continuous gradient [cPCO₂ - EtPCO₂] in mechanically ventilated patients.

First, warming the sensor to normal body temperature allowed to: 1) normalize the tonometry to physiologically compare the patients together; 2) largely limit the impact of arterial PCO₂ on cutaneous PCO₂, since the "arterialisation of the blood" was less than during warming between 42 to 44°C; 3) avoid the substraction of the metabolic constant (Hazinski and Severinghaus, 1982). In these conditions, cutaneous PCO₂ is significantly higher in shock patients compared to control patients (Fig. 1). This might result from an increase in tissue metabolism leading to higher CO₂ production or to a severe reduction in local blood flow washing the produced CO₂, or both. The difference with arterial PCO₂, or with the surrogate EtPCO₂, limits the impact of arterial PCO₂ and lung function, a reason to support the use of the gradient rather than cutaneous PCO₂ alone.

Second, the evolution of cPCO₂ gradients during 36 hours of intensive treatment differentiates survivors and non-survivors better than macro-circulation parameters (Fig. 3 and 4). These results are new since the use of transcutaneous PCO₂ measured at 42-44°C could not discriminate survivors and non-survivors in two recent studies (Yu et al., 2007; Yu et al., 2006). The use of these gradients are then relevant during shock state to predict outcome (Levy et al., 2003a; Weil, 2000). Cutaneous PCO₂ at normal body temperature better fits with tissue perfusion than macro-hemodynamic parameters nor oxygenation parameters.

Third, the cPCO₂ gradients ([cPCO₂ - aPCO₂] and [cPCO₂ - EtPCO₂]) can also be used as a monitor for treatment response, especially for blood volume as suggested by the effect of fluid challenge. Importantly, the reduction in PCO₂ gradients correlated well with the increase in microcirculatory skin blood flow during fluid challenge (Fig. 5).

Finally, even when arterial PCO₂ is not measured, the strict non-invasive gradient [cPCO₂ - EtPCO₂] can be used continuously to monitor the tissue perfusion (Fig. 1 to 5).

### REFERENCES

Creteur, J., Carollo, T., Soldati, G., Buchele, G., De Backer, D. and Vincent, J.L. (2007) The prognostic value of muscle St02 in septic patients. Intensive Care Med, 33, 1549-1556.
Creteur, J., De Backer, D., Sakr, Y., Koch, M. and Vincent, J.L. (2006) Sublingual capnometry tracks microcirculatory changes in septic patients. Intensive Care Med, 32, 516-523.
De Backer, D., Creteur, J., Preiser, J.C., Dubois, M.J. and Vincent, J.L. (2002) Microvascular blood flow is altered in patients with sepsis. Am J Respir Crit Care Med, 166, 98-104.
Dellinger, R.P., Levy, M.M., Carlet, J.M., Bion, J., Parker, M.M., Jaeschke, R., Reinhart, K., Angus, D.C., Brun-Buisson, C., Beale, R., Calandra, T., Dhainaut, J.F., Gerlach, H., Harvey, M., Marini, J.J., Marshall, J., Ranieri, M., Ramsay, G., Sevransky, J., Thompson, B.T., Townsend, S., Vender, J.S., Zimmerman, J.L. and Vincent, J.L. (2008) Surviving Sepsis Campaign: international guidelines for management of severe sepsis and septic shock: 2008. Intensive Care Med, 34, 17-60.
Eberhard, P. (2007) The design, use, and results of transcutaneous carbon dioxide analysis: current and future directions. Anesth Analg, 105, S48-52.
Eberhard, P., Gisiger, P.A., Gardaz, J.P. and Spahn, D.R. (2002) Combining transcutaneous blood gas measurement and pulse oximetry. Anesth Analg, 94, S76-80.
Fang, X., Tang, W., Sun, S., Huang, L., Chang, Y.T., Castillo, C. and Weil, M.H. (2006) Comparison of buccal microcirculation between septic and hemorrhagic shock. Crit Care Med, 34, S447-453.
Fries, M., Weil, M.H., Sun, S., Huang, L., Fang, X., Cammarata, G., Castillo, C. and Tang, W. (2006) Increases in tissue Pco2 during circulatory shock reflect selective decreases in capillary blood flow. Crit Care Med, 34, 446-452.
Gutierrez, G., Palizas, F., Doglio, G., Wainsztein, N., Gallesio, A., Pacin, J., Dubin, A., Schiavi, E., Jorge, M., Pusajo, J. and et al. (1992) Gastric intramucosal pH as a therapeutic index of tissue oxygenation in critically ill patients. Lancet, 339, 195-199.
Hazinski, T.A. and Severinghaus, J.W. (1982) Transcutaneous analysis of arterial PCO2. Med Instrum, 16, 150-153.
Heino, A., Hartikainen, J., Merasto, M.E., Alhava, E. and Takala, J. (1998) Systemic and regional pCO2 gradients as markers of intestinal ischaemia. Intensive Care Med, 24, 599-604.
Jin, X., Weil, M.H., Sun, S., Tang, W., Bisera, J. and Mason, E.J. (1998) Decreases in organ blood flows associated with increases in sublingual PCO2 during hemorrhagic shock. J Appl Physiol, 85, 2360-2364.
Kagawa, S., Otani, N., Kamide, M., Gisiger, P.A., Eberhard, P. and Severinghaus, J.W. (2004) Initial transcutaneous PCO2 overshoot with ear probe at 42 degrees C. J Clin Monit Comput, 18, 343-345.
Levy, B., Gawalkiewicz, P., Vallet, B., Briancon, S., Nace, L. and Bollaert, P.E. (2003a) Gastric capnometry with air-automated tonometry predicts outcome in critically ill patients. Crit Care Med, 31, 474-480.
Levy, M.M., Fink, M.P., Marshall, J.C., Abraham, E., Angus, D., Cook, D., Cohen, J., Opal; S.M., Vincent, J.L. and Ramsay, G. (2003b) 2001 SCCM/ESICM/ACCP/ATS/SIS International Sepsis Definitions Conference. Intensive Care Med, 29, 530-538.
Marik, P.E. and Bankov, A. (2003) Sublingual capnometry versus traditional markers of tissue oxygenation in critically ill patients. Crit Care Med, 31, 818-822.
Mindt, W., Eberhard, P. and Schafer, R. (1982) Monitoring of PCO2 by skin surface sensors. Biotelem Patient Monit, 9, 28-35.
Nakagawa, Y., Weil, M.H., Tang, W., Sun, S., Yamaguchi, H., Jin, X. and Bisera, J. (1998) Sublingual capnometry for diagnosis and quantitation of circulatory shock. Am JRespir Crit Care Med, 157,1838-1843.
Neviere, R., Mathieu, D., Chagnon, J.L., Lebleu, N., Millien, J.P. and Wattel, F. (1996) Skeletal muscle microvascular blood flow and oxygen transport in patients with severe sepsis. Am J Respir Crit Care Med, 153, 191-195.
Ristagno, G., Tang, W., Sun, S. and Weil, M.H. (2006) Role of buccal PCO2 in the management of fluid resuscitation during hemorrhagic shock. Crit Care Med, 34, S442-446.
Rooth, G., Ewald, U. and Caligara, F. (1987) Transcutaneous P02 and PCO2 monitoring at 37 degrees C. cutaneous P02 and PCO2. Adv Exp Med Biol, 220, 23-32.
Schabauer, A.M. and Rooke, T.W. (1994) Cutaneous laser Doppler flowmetry: applications and findings. Mayo Clin Proc, 69, 564-574.
Weil, M.H. (2000) Tissue PCO2 as universal marker of tissue hypoxia. Minerva Anestesiol, 66, 343-347.
Weil, M.H., Nakagawa, Y., Tang, W., Sato, Y., Ercoli, F., Finegan, R., Grayman, G. and Bisera, J. (1999) Sublingual capnometry: a new noninvasive measurement for diagnosis and quantitation of severity of circulatory shock. Crit Care Med, 27, 1225-1229.
Weil, M.H. and Sun, S. (2001) Tissue capnometry. Crit Care Med, 29, 460.
Yu, M., Chapital, A., Ho, H.C., Wang, J. and Takanishi, D., Jr. (2007) A prospective randomized trial comparing oxygen delivery versus transcutaneous pressure of oxygen values as resuscitative goals. Shock, 27, 615-622.
Yu, M., Morita, S.Y., Daniel, S.R., Chapital, A., Waxman, K. and Severino, R. (2006) Transcutaneous pressure of oxygen: a noninvasive and early detector of peripheral shock and outcome. Shock, 26, 450-456.

## Claims

1. A method for *ex vivo* obtaining an indicator of tissue perfusion in a patient, comprising calculating [cPCO₂ - aPCO₂] or [cPCO₂ - EtPCO₂], wherein cPCO₂ is the cutaneous partial pressure of carbon dioxide of said patient, measured with a carbon dioxide sensor without previous heating of said sensor at a temperature superior to 37.5°C, EtPCO₂ is the end-tidal partial pressure of carbon dioxide, and aPCO₂ is the arterial partial pressure of carbon dioxide of the same patient, wherein the result obtained is an indicator of tissue perfusion.

2. The method of claim 1, wherein the carbon dioxide sensor used for measuring cPO₂ has been placed on the ear lobe or on the scalp of said patient.

3. The method of claim 1 or claim 2, wherein the carbon dioxide sensor used for measuring cPO₂ has been applied to the skin of said patient without previous heating.

4. A method for assessing perfusion failure of a patient, comprising:
(i) obtaining an indicator of tissue perfusion by a the method according to any of claims 1 to 3; and
(ii) comparing the obtained result with a predetermined threshold;
wherein if the result obtained in step (i) is superior to said predetermined threshold, it is indicative of perfusion failure in the patient.

5. The method of claim 4, wherein said predetermined threshold is 9 mmHg for [cPCO₂ - aPCO₂] and said predetermined threshold is 15 mmHg for [cPCO₂ - EtPCO₂].

6. A method for *ex vivo* performing a continuous and non-invasive hemodynamic monitoring of a patient, comprising:
(i) obtaining [cPCO₂ - EtPCO₂] by a the method according to any of claims I to 3; and
(ii) observing the evolution of [cPCO₂ - EtPCO₂], wherein an increase of [cPCO₂ - EtPCO₂] indicates a deterioration of the hemodynamic state of the patient, and a decrease of [cPCO₂ - EtPCO₂] indicates an amelioration of the hemodynamic state of the patient.

7. A method for *ex vivo* predicting the outcome of a patient in septic shock, comprising:
(i) obtaining an indicator of tissue perfusion of said patient by a the method according to any of claims 1 to 3;
(ii) repeating step (i) at least one time ;
(iii) comparing the results obtained in the preceding steps;
wherein a decrease of [cPCO₂ - EtPCO₂] and/or [cPCO₂ - aPCO₂] during the 24 hours following the beginning of the treatment of septic shock is indicative of a good prognosis, and the absence of decrease is indicative of a bad prognosis.

8. The method of claim 7, wherein [cPCO₂ - EtPCO₂] ≥ 25 mm Hg, or [cPCO₂ - aPCO₂] ≥ 16 mm Hg 24 hours after the beginning of the treatment of septic shock is indicative of a high risk of death.

9. A device for performing a method according to any of claims 1 to 8, comprising:
a first carbon dioxide sensor for detecting a partial pressure of carbon dioxide (PCO₂), the sensor being adapted for being attached to the ear lobe and/or the scalp and measuring the cutaneous PCO₂ (cPCO₂) of a patient without previous heating of said sensor at a temperature superior to 37.5°C;
a second carbon dioxide sensor for detecting a partial pressure of carbon dioxide (PCO₂), the sensor being adapted for measuring the end-tidal PCO₂ (EtPCO₂) of an intubated patient; and
a computer operably connected to both sensors, wherein said computer calculates the difference between the partial pressures of carbon dioxide measured by said sensors.

10. The device of claim 9, which further comprises a third carbon dioxide sensor, said third sensor being adapted for measuring the arterial PCO₂ (aPCO₂).

11. The device of claims 9 and 10, further comprising indicating means operably connected to the computer, wherein the indicating means indicates a degree of perfusion of the patient associated with the detected partial pressures of carbon dioxide.

## Patentansprüche

1. Ein Verfahren zum *ex vivo* Erhalten eines Indikators der Gewebeperfusion in einem Patienten, umfassend Berechnen [cPCO₂ - aPCO₂] oder [cPCO₂-EtPCO₂], wobei cPCO₂ der kutane Kohlendioxidpartialdruck des Patienten ist, gemessen mit einem Kohlendioxidsensor ohne vorheriges Erwärmen des Sensors auf eine Temperatur oberhalb von 37,5 °C, EtPCO₂ der endtidale Partialdruck des Kohlendioxids ist und aPCO₂ der arterielle Partialdruck des Kohlendioxids desselben Patienten ist, wobei das erhaltene Ergebnis ein Indikator der Gewebeperfusion ist.

2. Das Verfahren nach Anspruch 1, wobei der Kohlendioxidsensor, der für die Messung von cPO₂ verwendet wird, auf dem Öhrläppchen oder der Kopfhaut des Patienten angebracht wird.

3. Das Verfahren nach Anspruch 1 oder 2, wobei der Kohlendioxidsensor, der für die Messung von cPO₂ verwendet wird, auf der Haut des Patienten ohne vorherige Erwärmung angelegt wird.

4. Ein Verfahren zum Beurteilen eines Perfusionsversagens eines Patenten, welches umfasst:
(i) Erhalten eines Indikators einer Gewebeperfusion gemäß einem Verfahren nach irgendeinem der Ansprüche 1 bis 3; und
(ii) Vergleichen des erhaltenen Ergebnisses mit einem vorbestimmten Schwellwert;
wobei, wenn das in Schritt (i) erhaltene Ergebnis oberhalb des vorbestimmten Schwellwerts liegt, es ein Perfusionsversagen in dem Patienten anzeigt.

5. Das Verfahren nach Anspruch 4, wobei der vorbestimmte Schwellwert 9 mmHg für [cPCO₂ - aPCO₂] beträgt und der vorbestimmte Schwellwert 15 mmHg für [cPCO₂ - EtPCO₂] beträgt.

6. Ein Verfahren zur *ex vivo* Durchführung einer kontinuierlichen und nichtinvasiven hämodynamischen Überwachung eines Patienten, welches umfasst:
(i) Erhalten von [cPCO₂ - EtPCO₂] durch ein Verfahren gemäß irgendeinem der Ansprüche 1 bis 3; and
(ii) Beobachten der Entwicklung von [cPCO₂ - EtPCO₂], wobei ein Anstieg von [cPCO₂ - EtPCO₂] eine Verschlechterung des hämodynamischen Zustands des Patienten anzeigt und ein Abfall von [cPCO₂ - ETPCO₂ eine Verbesserung des hämodynamischen Zustands des Patienten anzeigt.

7. Ein Verfahren zur *ex vivo* Vorhersage des Ausgangs eines septischen Schocks bei einem Patienten, welches umfasst:
(i) Erhalten eines Indikators der Gewebeperfusion des Patienten durch ein Verfahren gemäß irgendeinem der Ansprüche 1 bis 3;
(ii) wenigstens einmaliges Wiederholen von Schritt (i);
(iii) Vergleichen der in den vorherigen Schritten erhaltenen Ergebnisse;
wobei ein Abfall von [cPCO₂ - EtPCO₂] und/oder [cPCO₂ - aPCO₂] innerhalb von 24 Stunden nach dem Behandlungsbeginn des septischen Schocks eine gute Prognose anzeigt, während die Abwesenheit eines Abfalls eine schlechte Prognose anzeigt.

8. Das Verfahren nach Anspruch 7, wobei [cPCO₂- EtPCO₂] ≥ 25 mmHg oder [cPCO₂- aPCO₂] ≥ 16 mmHg 24 Stunden nach dem Behandlungsbeginn des septischen Schocks ein großes Sterberisiko anzeigt.

9. Eine Vorrichtung zur Durchführung eines Verfahrens gemäß irgendeinem der Ansprüche 1 bis 8 mit:
einem ersten Kohlendioxidsensor zum Erfassen eines Partialdrucks von Kohlendioxid (PCO₂), wobei der Sensor ausgelegt ist, um an dem Ohrläppchen und/oder der Kopfhaut angebracht zu werden, und zum Messen des kutanen PCO₂ (cPCO₂) eines Patienten ohne vorheriges Erwärmen des Sensors auf eine Temperatur oberhalb von 37,5 °C;
einem zweiten Kohlendioxidsensor zum Erfassen eines Partialdrucks des Kohlendioxids (PCO₂), wobei der Sensor ausgelegt ist zum Messen des endtidalen PCO₂ (EtPCO₂) eines intubierten Patienten; und
einem Computer, der operativ mit beiden Sensoren verbunden ist, wobei der Computer die Differenz zwischen den Partialdrücken des Kohlendioxids, welche durch die Sensoren gemessen werden, berechnet.

10. Die Vorrichtung nach Anspruch 9, welche ferner einen dritten Kohlendioxidsensor umfasst, wobei der dritte Sensor zum Messen des arteriellen PCO₂ (aPCO₂) ausgelegt ist.

11. Die Vorrichtung nach den Ansprüchen 9 und 10, ferner umfassend Anzeigemittel, welche operativ mit dem Computer verbunden sind, wobei die Anzeigemittel den Perfusionsgrad des Patienten, verbunden mit den erfassten Partialdrücken des Kohlendioxids, anzeigen.

## Revendications

1. Procédé d'obtention *ex vivo* d'un indicateur de la perfusion tissulaire chez un patient, comprenant le calcul de [cPCO₂ - aPCO₂] ou [cPCO₂ - EtPCO₂], dans lequel cPCO₂ est la pression partielle cutanée de dioxyde de carbone dudit patient mesurée avec un capteur de dioxyde de carbone sans chauffage préalable dudit capteur à une température supérieure à 37,5 °C, EtPCO₂ est la pression partielle de dioxyde de carbone en fin d'expiration, et aPCO₂ est la pression artérielle partielle de dioxyde de carbone du même patient, dans lequel le résultat obtenu est un indicateur de la perfusion tissulaire.

2. Procédé selon la revendication 1, dans lequel le capteur de dioxyde de carbone utilisé pour mesurer la cPCO₂ est placé sur le lobe de l'oreille ou le cuir chevelu dudit patient.

3. Procédé selon la revendication 1 ou la revendication 2, dans lequel le capteur de dioxyde de carbone utilisé pour mesurer la cPCO₂ est appliqué sur la peau dudit patient sans chauffage préalable.

4. Procédé d'évaluation d'une insuffisance de perfusion chez un patient, comprenant :
(i) l'obtention d'un indicateur de perfusion tissulaire par le procédé selon l'une quelconque des revendications 1 à 3 ; et
(ii) la comparaison du résultat obtenu à un seuil prédéterminé ;
dans lequel si le résultat obtenu dans l'étape (i) est supérieur audit seuil prédéterminé, cela indique une insuffisance de perfusion chez le patient.

5. Procédé selon la revendication 4, dans lequel ledit seuil prédéterminé est de 9 mmHg pour [cPCO₂ - aPCO₂] et ledit seuil prédéterminé est de 15 mmHg pour [cPCO₂ - EtPCO₂].

6. Procédé de réalisation *ex vivo* d'une surveillance hémodynamique continue et non invasive d'un patient, comprenant :
(i) l'obtention, de [cPCO₂ - EtPCO₂] par un procécé selon l'une quelconque des revendications 1 à 3 ; et
(ii) l'observation de l'évolution de [cPCO₂ - EtPCO₂], dans lequel une augmentation de [cPCO₂ - EtPCO₂] indique une détérioration de l'état hémodynamique du patient, et une diminution de [cPCO₂ - EtPCO₂] indique une amélioration de l'état hémodynamique du patient.

7. Procédé de prédiction *ex vivo* de l'évolution d'un patient après un choc septique, comprenant :
(i) l'obtention d'un indicateur de perfusion tissulaire dudit patient par le procédé selon l'une quelconque des revendications 1 à 3 ;
(ii) la répétition de l'étape (i) au moins une fois ;
(iii) la comparaison des résultats obtenus dans les étapes précédentes ;
dans lequel une réduction de [cPCO₂ - EtPCO₂] et/ou [cPCO₂ - aPCO₂] pendant les 24 heures suivant le début du traitement d'un choc septique indique un bon prognostic, et l'absence de réduction indique un mauvais pronostic.

8. Procédé selon la revendication 7, dans lequel [CPCO₂ - EtPCO₂] ≥ 25 mmHg, ou [cPCO₂ - aPCO₂] ≥ 16 mmHg 24 heures après le début du traitement du choc septique indique un risque élevé de décès.

9. Dispositif permettant de mettre en oeuvre un procédé selon l'une quelconque des revendications 1 à 8, comprenant :
un premier capteur de dioxyde de carbone pour détecter une pression partielle de dioxyde de carbone (PCO₂), le capteur étant adapté pour être fixé au lobe de l'oreille et/ou au cuir chevelu et pour mesurer la PCO₂ cutanée (cPCO₂) d'un patient sans chauffage préalable dudit capteur à une température supérieure à 37,5 °C ;
un second capteur de dioxyde de carbone pour détecter une pression partielle de dioxyde de carbone (PCO₂), le capteur étant adapté pour mesurer la PCO₂ de fin d'expiration (EtPCO₂) d'un patient intubé ; et
un ordinateur opérationnellement connecté aux deux capteurs, dans lequel ledit ordinateur calcule la différence entre les pressions partielles de dioxyde de carbone mesurées par lesdits capteurs.

10. Dispositif selon la revendication 9, qui comprend en outre un troisième capteur de dioxyde de carbone, ledit troisième capteur étant adapté pour mesurer la PCO₂ artérielle (aPCO₂).

11. Dispositif selon les revendications 9 et 10, comprenant en outre un moyen d'indication opérationnellement raccordé à l'ordinateur, dans lequel le moyen d'indication indique un degré de perfusion du patient associé aux pressions partielles de dioxyde de carbone détectées.
